# EUROPEAN PATENT SPECIFICATION

(11) **EP 0 350 042 B1**
(45) Date of publication and mention of the grant of the patent: **27.04.1994**
(21) Application number: 89112384.6
(22) Date of filing: 06.07.1989
(51) Int. Cl.: C07D 271/06, C07D 251/06, C07C 239/20, C10M 105/70, C09K 5/00

(54) **Fluorinated compounds and process for preparing them**
Fluorierte Verbindungen und Verfahren zu ihrer Herstellung
Composés fluorurés et procédé pour leur préparation

(30) Priority: 07.07.1988 IT 2126688
(43) Date of publication of application: 10.01.1990
(73) Proprietor: AUSIMONT S.p.A., I-20121 Milano (IT)
(72) Inventor: Malacrida, Alessandro, I-20050 Sovico Milano (IT); Desmarteau, Darryl D., Clemson South Carolina 29631 (US)
(74) Representative: Barz, Peter, Dr.

(56) References cited:
- EP-A- 0 010 452
- US-A- 3 214 465
- US-A- 4 010 212
- US-A- 4 605 737
- JOURNAL OF THE CHEMICAL SOCIETY, 1955, Barr and Haszeldine : "Perfluoroalkyl Derivatives of Nitrogen . Part I.", pp. 1885-1886
- CHEMICAL ABSTRACTS, vol. 93, no. 7, August 18, 1980, abstract 70905t
- Journal of Fluorine Chemistry, vol. 15 (1980), pp. 183-189

## Description

The present invention relates to new fluorinated compounds and to a process for preparing them.

More particularly, it relates to three fluorinated compounds obtainable by reacting 3,3,4,4-tetrafluoro-2-(trifluoromethyl)-1,2-oxazetidine: 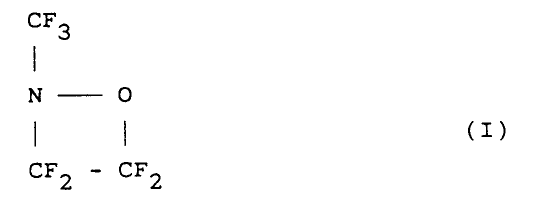
with a Lewis acid.

The above oxazetidine of formula (I) is a known compound, described by D.A. Barr et al. in J. Chem. Soc. 1955, 1881.

It has now, surprisingly, been found that said compound reacts with Lewis acids, thereby generating three fluorinated compounds.

An object of the present invention is to provide the above fluorinated compounds.

Another object is to provide a process for preparing them.

The first object is achieved by the compounds of the following three formulae: 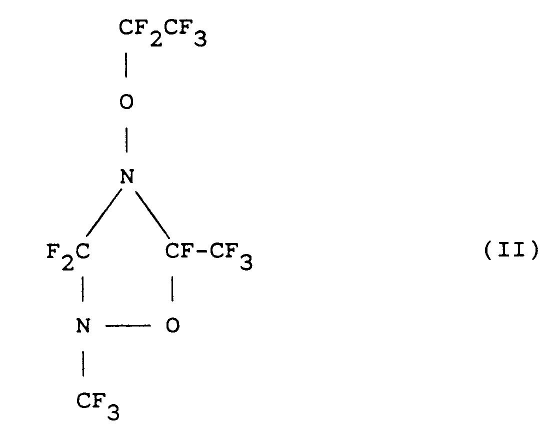
3,3,5-trifluoro-2,5-di(trifluoromethyl)-4-(1,1,2,2,2-pentafluoroethoxy)-1,2,4-oxadiazolidine; 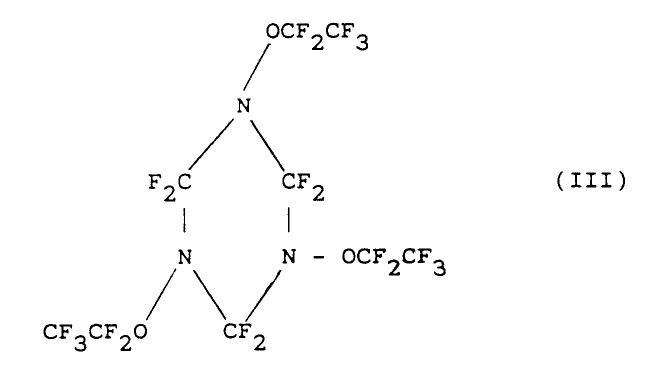
2,2,4,4,6,6-hexafluoro-1,3,5-tris(1,1,2,2,2-pentafluoroethoxy)-1,3,5-triazine;

(CF₃)₂N-O-CF₂CF₃ (IV)

N,N-bis(trifluoromethyl)-N-(1,1,2,2,2-pentafluoroethoxy)amine.

The compound of formula (II), which is a liquid at atmospheric pressure with a boiling point of 75°C is, for example, useful as lubricant, heat transfer medium and test fluid for electronic components.

The compound of formula (III), a solid compound with a melting point of 60 to 61°C is, for example, useful as lubricant.

The compound for formula (IV), a low-boiling liquid with a boiling point of 27°C may, for example, be used as test fluid for electronic components and as heat transfer medium.

The above compounds are formed simultaneously in the process according to the present invention. This process is characterized in that 3,3,4,4-tetrafluoro-2-(trifluoromethyl)-1,2-oxazetidine of formula (I) is reacted with a Lewis acid.

The reaction is usually carried out at a temperature of from about 0 to about 100°C, and preferably of from about 10 to about 40°C.

As Lewis acid fluorinated Lewis acids, in particular SbF₅ and AsF₅, are preferably used.

The molar ratio of the Lewis acid (for instance SbF₅) to oxazetidine (I) generally ranges from 0.1 to 2.0 and preferably from 0.9 to 1.1.

The reaction can be carried out batchwise inside an autoclave under the autogenous pressure of the system. The reaction time generally ranges from 1 minute up to 24 hours.

At the end of the reaction the reaction mixture is preferably treated with a neutralizing agent in order to neutralize the Lewis acid (for example, SbF₅).

A particularly suitable neutralizing agent is NaF. The molar ratio of neutralizing agent to Lewis acid (for example, NaF to SbF₅) usually is within the range of from about 1 to about 50 and preferably of from 5 to 15.

The following example is given in order to illustrate the present invention.

### EXAMPLE

Inside a 20 ml tubular reactor of Teflon^{(R)} FEP, equipped with a valve, the following compounds were condensed, in the order given, at the temperature of liquid nitrogen (-196°C):
2.88 g (13.3 mmol) of SbF₅; and
2.90 g (14.6 mmol) of oxazetidine (I).

Said oxazetidine had been prepared by reacting CF₃-N=O with CF₂=CF₂ (D.A. Barr et al., J. Chem. Soc. 1955, 1881).

The reactor then was kept at 20°C for 12 hours.

To the crude reaction mixture, cooled down to -196°C and kept under an anhydrous nitrogen atmosphere, 5.60 g 133 mmol) of fine NaF powder were added.

Thereafter, the reactor was kept at 20°C for a further 24 hours and was repeatedly shaken in order to achieve a good solid-liquid contact.

The reaction products were recovered by transfering them under low pressure (10⁻³ torr) from the reactor into a cold trap (temperature -196°C).

This step required 24 hours, whereupon the mixture of the reaction products was distilled under a pressure of 10⁻³ torr.

The vapours emerging from the distillation flask were passed through four cold traps maintained at temperatures of -50°C, -85°C, -110°C and -196°C, respectively.

Inside the trap at -50°C, 145 mg of compound of formula (III) were collected (molar yield 5% relative to oxazetidine (I)).

Said compound of formula (III) was characterized by the following spectra:
Mass spectrum:
- chemical ionization (CI)
- CI gas = CH₄
m/z, 598 (MH⁺) 578 (MH-HF⁺)
Infrared spectrum:
- main absorption bands (cm⁻¹)
1391 (d) 1332 (d) 1289 (f) 1242 (f)
1180 (m) 1089 (f) 946 (m)
¹⁹F-N.M.R. spectrum:
(internal reference: CFCl₃; solvent: CDCl₃; temperature = 50°C). 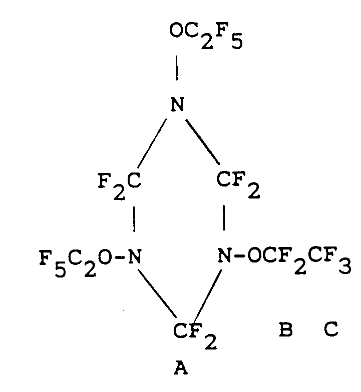
- A =: -84.7 ppm

- B =: -93.0 ppm
- C =: -84.7 ppm.

Inside the trap at -85°C, 1.37 g of compound of formula (II) were collected (molar yield 47% relative to oxazetidine (I)).

Said compound was characterized by the following spectra:
Mass spectrum:
- chemical ionization (CI)
- CI gas = CH₄
m/z, 399 (MH⁺) 379 (MH-HF⁺).
Infrared spectrum:
- main absorption bands (cm⁻¹)
1362 (d) 1315 (m) 1282 (m) 1246(f) 1225 (f) 1179 (m) 1120 (m) 1098 (f) 942 (d).
¹⁹F-N.M.R. spectrum:

(internal reference: CFCl₃; solvent: CDCl₃) 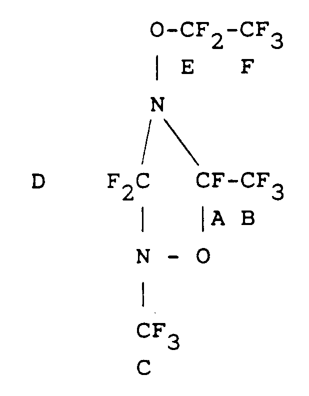
- A =: -111.6 ppm
- B =: - 82.3 ppm
- C =: - 66.7 ppm
- D =: - 78.7 ppm; -83.1 ppm (AB system)
- E =: - 90.1 ppm; -95.2 ppm (AB system)
- F =: - 84.7 ppm.

Inside the trap at -110°C, 0.42 g of compound of formula (IV) were collected (molar yield 10% relative to oxazetidine (I)).

Said compound was characterized by the following spectra:
Mass spectrum:
- chemical ionization (CI)
- CI gas = CH₄
- m/z, 288 (MH⁺) 287 (M⁺) 258 (M-F⁺).
Infrared spectrum:
- main absorption bands (cm⁻¹)
1361 (d) 1319 (f) 1272 (f) 1240 (f) 1218 (f) 1183 (f) 1095 (f) 1035 (d) 970 (m) 713 (m).
¹⁹F-N.M.R. spectrum:
(internal reference: CFCl₃; solvent; CDCl₃) 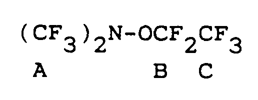
- A =: -67.9 ppm
- B =: -94.3 ppm
- C =: -85.0 ppm.

The trap at -196°C contained 0.25 g of oxazetidine of formula (I).

## Claims

1. Process for the simultaneous preparation of 3,3,5-trifluoro-2,5-di(trifluoromethyl)-4-(1,1,2,2,2-penta-fluoroethoxy)-1,2,4-oxadiazolidine of formula (II): 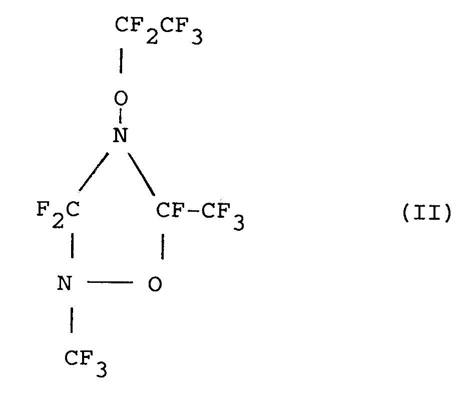 2,2,4,4,6,6-hexafluoro-1,3,5-tris(1,1,2,2,2-pentafluoroethoxy)-1,3,5-triazine of formula (III): 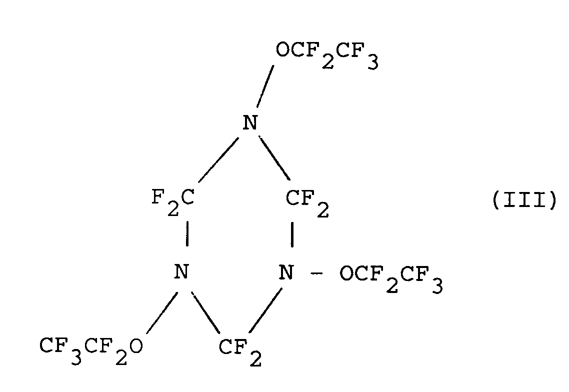 and N,N-bis(trifluoromethyl)-N-(1,1,2,2,2-pentafluoroethoxy)-amine of formula (IV):
(CF₃)₂N-O-CF₂CF₃ (IV)
characterized in that 3,3,4,4-tetrafluoro-2-(trifluoromethyl)-1,2-oxazetidine of formula (I): 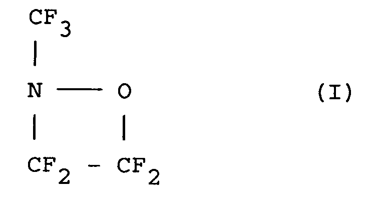 is reacted with a Lewis acid.

2. Process according to claim 1, characterized in that the reaction is carried out at a temperature of from 0°C to 100°C.

3. Process according to claim 1 or 2, characterized in that the Lewis acid is a fluorinated compound.

4. Process according to any one of claims 1 to 3, characterized in that the Lewis acid is selected from SbF₅, AsF₅ and mixtures thereof.

5. Process according to claim 4, characterized in that the Lewis acid is SbF₅.

6. Compound of formula (II) obtainable by the process of claim 1, i.e., 3,3,5-trifluoro-2,5-di(trifluoromethyl)-4-(1,1,2,2,2-penta-fluoroethoxy)-1,2,4-oxadiazolidine.

7. Compound of formula (III) obtainable by the process of claim 1, i.e., 2,2,4,4,6,6-hexafluoro-1,3,5-tris(1,1,2,2,2-pentafluoroethoxy)-1,3,5-triazine.

8. Compound of formula (IV) obtainable by the process of claim 1, i.e., N,N-bis(trifluoromethyl)-N-(1,1,2,2,2-pentafluoroethoxy)amine.

9. Use of the compounds according to any one of claims 6 and 7 as lubricants.

10. Use of the compounds according to any one of claims 6 and 8 as heat transfer media or test fluids for electronic compounds.

## Patentansprüche

1. Verfahren zur gleichzeitigen Herstellung von 3,3,5-Trifluor-2,5-di(trifluormethyl)-4-(1,1,2,2,2-Pentafluorethoxy)-1,2,4-oxadiazolidin der Formel (II): 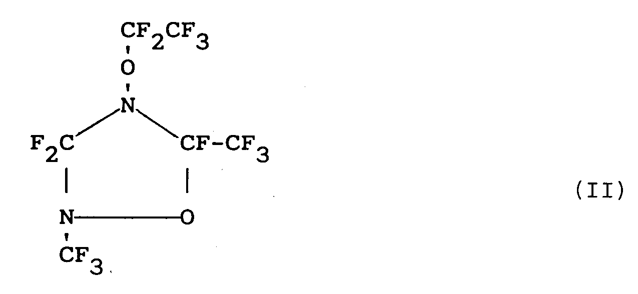 2,2,4,4,6,6-Hexafluor-1,3,5-tris(1,1,2,2,2-pentafluorethoxy)-1,3,5-triazin der Formel (III): 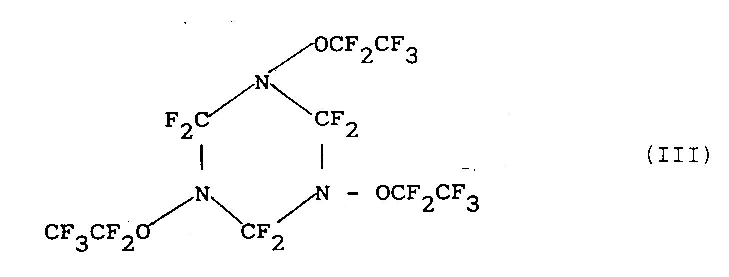 und N,N-Bis(trifluormethyl)-N-(1,1,2,2,2-pentafluorethoxy)amin der Formel (IV):
(CF₃)₂N-O-CF₂CF₃ (IV)
dadurch gekennzeichnet, daß 3,3,4,4-Tetrafluor-2-(trifluormethyl)-1,2-oxazetidin der Formel (I): 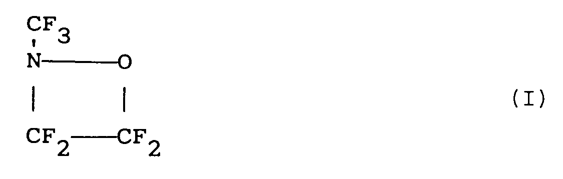 mit einer Lewis-Säure umgesetzt wird.

2. Verfahren nach Anspruch 1, dadurch gekennzeichnet, daß die Reaktion bei einer Temperatur von 0°C bis 100°C durchgeführt wird.

3. Verfahren nach Anspruch 1 oder 2, dadurch gekennzeichnet, daß die Lewis-Säure eine fluorierte Verbindung ist.

4. Verfahren nach irgendeinem der Ansprüche 1 bis 3, dadurch gekennzeichnet, daß die Lewis-Säure aus SbF₅, AsF₅ und Mischungen davon ausgewählt wird.

5. Verfahren nach Anspruch 4, dadurch gekennzeichnet, daß die Lewis-Säure SbF₅ ist.

6. Verbindung der Formel (II), erhältlich durch das Verfahren von Anspruch 1, d.h. 3,3,5-Trifluor-2,5-di(trifluormethyl)-4-(1,1,2,2,2-pentafluorethoxy)-1,2,4-oxadiazolidin.

7. Verbindung der Formel (III), erhältlich durch das Verfahren von Anspruch 1, d.h. 2,2,4,4,6,6-Hexafluor-1,3,5-tris(1,1,2,2,2-pentafluorethoxy)-1,3,5-triazin.

8. Verbindung der Formel (IV), erhältlich durch das Verfahren von Anspruch 1, d.h. N,N-Bis(trifluormethyl)-N-(1,1,2,2,2-pentafluorethoxy)amin.

9. Verwendung der Verbindungen nach irgendeinem der Ansprüche 6 und 7 als Schmiermittel.

10. Verwendung der Verbindungen nach irgendeinem der Ansprüche 6 und 8 als Wärmeübertragungsmedien oder Testfluide für elektronische Komponenten.

## Revendications

1. Procédé pour la préparation simultanée de 3,3,5-trifluoro-2,5-di(trifluorométhyl)-4-(1,1,2,2,2-pentafluoroéthoxy)-1,2,4-oxadiazolidine de formule (II): 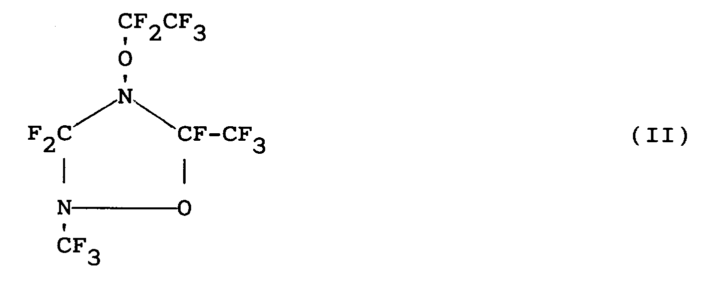 2,2,4,4,6,6-hexafluoro-1,3,5-tris(1,1,2,2,2-pentafluoroéthoxy)-1,3,5-triazine de formule (III): 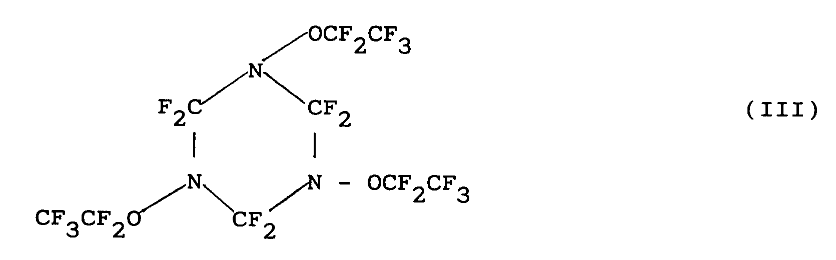 et de N,N-bis(trifluorométhyl)-N-(1,1,2,2,2-pentafluoroéthoxy)amine de forme IV :
(CF₃)₂N-O-CF₂CF₃ (IV)
caractérisé en ce qu'une 3,3,4,4-tétrafluoro-2-trifluorométhyl)-1,2-oxazétidine de formule (I): 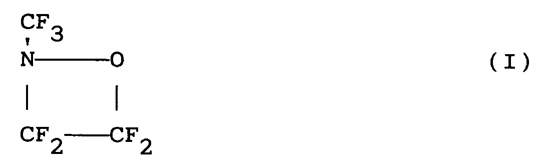 réagit avec un acide de Lewis.

2. Procédé selon la revendication 1, caractérisé en ce que la réaction est effectuée à une température comprise entre 0°C et 100°C.

3. Procédé selon la revendication 1 ou 2, caractérisé en ce que l'acide de Lewis est un composé fluoré.

4. Procédé selon l'une quelconque des revendications 1 à 3, caractérisé en ce que l'acide de Lewis est choisi dans le groupe comprenant SbF₅, AsF₅ et leurs mélanges.

5. Procédé selon la revendication 4, caractérisé en ce que l'acide de Lewis est SbF₅.

6. Composé de formule (II) qui peut être obtenu par le procédé selon la revendication 1, consistant en 3,3,5-trifluoro-2,5-di(trifluorométhyl)-4-(1,1,2,2,2-pentafluoroéthoxy)-1,2,4-oxadiazolidine.

7. Composé de formule (III) qui peut être obtenu par le procédé selon la revendication 1, consistant en 2,2,4,4,6,6-hexafluoro-1,3,5-tris(1,1,2,2,2-pentafluoroéthoxy)-1,3,5-triazine;

8. Composé de formule (IV) qui peut être obtenu par le procédé selon la revendication 1, consistant en N,N-bis(trifluorométhyl)-N-(1,1,2,2,2-pentafluoroéthoxy)amine.

9. Utilisation des composés selon l'une quelconque des revendications 6 et 7, en tant que lubrifiants.

10. Utilisation des composés selon l'une quelconque des revendications 6 et 8, en tant que milieu caloporteur ou fluide de test pour composants électroniques.
